# EUROPEAN PATENT SPECIFICATION

(11) **EP 3 411 053 B1**
(45) Date of publication and mention of the grant of the patent: **05.08.2020**
(21) Application number: 17719705.0
(22) Date of filing: 10.04.2017
(51) Int. Cl.: A61K 36/19, A61P 7/02

(54) **ANTIPLATELET ACTIVITY OF THEACANTHUS MOLLIS**
ANTIPLÄTTCHENAKTIVITÄT VON THEACANTHUS MOLLIS
ACTIVITÉ ANTIPLAQUETTAIRE DE L'EXTRAIT TOTAL DE GRAINES D'ACANTHUS MOLLIS

(43) Date of publication of application: 12.12.2018
(73) Proprietor: Tselepis, Alexandros, 45 445 Ioannina (GR); Anagnostopoulos, Spyridon, 67100 Kimmeria Xanthi (GR); Georgitsopoulos, Dimitrios, 17 564 Palaio Faliro Attikis (GR)
(72) Inventor: Tselepis, Alexandros, 45 445 Ioannina (GR); Anagnostopoulos, Spyridon, 67100 Kimmeria Xanthi (GR); Georgitsopoulos, Dimitrios, 17 564 Palaio Faliro Attikis (GR)
(86) International application number: PCT/GR2017/000019
(87) International publication number: WO 2018/189559

(56) References cited:
- EP-A2- 1 736 167
- GIANLUCA CAMPO ET AL: "Platelet aggregation values in patients with cardiovascular risk factors are reduced by verbascoside treatment. A randomized study", PHARMACOLOGICAL RESEARCH., vol. 97, 1 July 2015 (2015-07-01), pages 1-6, XP055429015, GB ISSN: 1043-6618, DOI: 10.1016/j.phrs.2015.03.020
- GIANLUCA CAMPO ET AL: "The in vitro effects of verbascoside on human platelet aggregation", JOURNAL OF THROMBOSIS AND THROMBOLYSIS, KLUWER ACADEMIC PUBLISHERS, BO, vol. 34, no. 3, 22 June 2012 (2012-06-22), pages 318-325, XP035117721, ISSN: 1573-742X, DOI: 10.1007/S11239-012-0757-Z
- Etagegnehu Assefa ET AL: "Iridoid glycosides from the root of Acanthus sennii", Journal of Pharmacy & Pharmacognosy Research, 1 December 2016 (2016-12-01), pages 231-237, XP055429130, Retrieved from the Internet: URL:http://jppres.com/jppres/pdf/vol4/jppr es16.141_4.6.231.pdf
- PAWADEE NOIARSA ET AL: "Acanmontanoside, a New Phenylethanoid Diglycoside from Acanthus montanus", MOLECULES, vol. 15, no. 12, 7 December 2010 (2010-12-07), pages 8967-8972, XP055429025, DOI: 10.3390/molecules15128967
- AMMAR BADER ET AL: "Modulation of Cox-1, 5-, 12- and 15-Lox by Popular Herbal Remedies Used in Southern Italy Against Psoriasis and Other Skin Diseases : MODULATION OF EICOSANOID SYNTHESIS BY ITALIAN ANTIPSORIATIC PLANTS", PHYTOTHERAPY RESEARCH., vol. 29, no. 1, 3 October 2014 (2014-10-03), pages 108-113, XP055428992, GB ISSN: 0951-418X, DOI: 10.1002/ptr.5234 cited in the application

## Description

### FIELD OF THE INVENTION

The present invention relates to the antiplatelet activity expressed by the total extract of *Acanthus mollis* seeds as well by each one of its constituents DIBOA-Glc, Verbascoside and Isoverbascoside as well as their combination.

### BACKGROUND OF THE INVENTION

Nowadays atherothrombosis is the leading cause of death worldwide. Platelet aggregation plays an important role in the pathophysiology of artery and venous thrombosis, while platelet activation also significantly contributes to atherogenesis and other pathophysiological conditions such as inflammation and carcinogenesis. Therefore, antiplatelet therapy represents the cornerstone for the treatment of patients with cardiovascular disease (CVD) and particularly patients presenting an acute coronary syndrome (ACS). Furthermore, long-term treatment of patients with antiplatelet drugs may significantly contribute to the prevention of other disorders such as cancer. Currently available antiplatelet drugs target key points of platelet activation such as the synthesis of thromboxane A₂ (TxA₂) via cyclooxygenase-1 (COX-1), the P2Y₁₂ receptor of Adenosine Diphosphate (ADP) and the integrin-receptor α_{IIb}/β₃ (GPIIb/IIIa). Current research is directed towards the development of antagonists targeting other platelet receptors such as the thrombin receptor, named as Protease Activated Receptor-1 (PAR-1) *(*Michelson AD, et al. Nat Rev Drug Discov. 2010;9:154-69*;* Tsoumani ME, Tselepis AD, Curr Pharm Des. 2017*;Epub ahead of print*).

The last years, particular interest in current research have natural products (olive oil, mushrooms, wine, etc.) and their constituents which exhibit several biological activities such as anti-inflammatory, antioxidant and antiplatelet *(*Kontogianni VG, et al. J. Agric. Food Chem. 2016;22:4511-4521*;* Tzakos AG, et al. J Agric Food Chem. 2012;60:6977-83*;* Jose N, et al. Phytother. Res. 2004;1:43-6). Therefore, the aim of the present research was to study the possible antiplatelet effect of *Acanthus mollis* seeds' total extract and to identify the main components responsible for the expression of this effect.

*Acanthaceae* is a large family of plants comprising 4.300 species categorized to 346 genera (Rezanka T, et al. Phytochem. 2009;70:1049-1054). The members of this family are found in tropical and temperate regions, mainly in the Mediterranean region. *Acanthus* is a genus of wild flowers which grows in meadows, forests and rocky and bushy hills. The most important representative of this genus is *Acanthus mollis,* an herbaceous, perennial plant that is found in various Mediterranean regions, from Portugal and North-West Africa to the Balkan countries. The plant germinates only during the spring and early summer. However, it is described as multiannual because the rhizome remains alive throughout the year. The main foliage is formed by lobed, slightly thorny, glossy, green leaves. A special feature of the leaves is their soft texture as they do not have thorns or fluff. The flowers are white with purple color. The fruit of the plant has a smooth surface, its size resembles that of the lemon and its color is green. As it matures, it hardens breaks and eliminates the seeds that it contains. The seeds are also hard, have brown color, a slightly crumpled coat and their shape is oval and flat. The isolation and characterization of various compounds including verbascoside, isoverbascoside and DIBOA-Glc form several Acanthus species including *Acanthus montanus* and *Acanthus sennii,* has been previously described (Noiarsa P, et al. Molecules. 2010;15:8967-8972*;* Assefa et al. J Pharm Pharmacogn Res. 2016;4:231-237*).* The above phytochemicals also exist in *Acanthus mollis. Acanthus mollis* is used against psoriasis and other skin diseases that occur with an imbalanced production of eicosanoids. In a previous study it was demonstrated that the methanol leaves' extract inhibits the 5- lipoxygenase (5-LOX) and cyclooxygenase- 1 (COX-1) activities at a concentration of 200 mg / mL and increases the biosynthesis of 15 (S) -HETE, an anti-inflammatory eicosanoid (Bader A, et al. Phytother. Res. 2015;29:108-113*).* In a previously published patent (*Patent No* EP 1 736 167 A2), the antiaggregatory effect of verbascosite and isoverbascosite (these 2 substances were studied separately) isolated form *Syringa vulgaris* extract 1, was studied in rabbit platelets which have similarities but also marked differences as concern to their aggregation compared with human platelets (Packham MA, et al. Comp Biochem Physiol Comp Physiol. 1992;103:35-54*,* Harfenist EJ, et al. Blood 1988;71:132-136*,* Moyle M, et al. Blood. 1989;74: 92a*,* Verhallen P and Barth M. Thromb Haemost. 1991; 65:1144*).*

The antiplatelet effect of verbascoside obtained by cell line of a *Syringa vulgaris* plant leaves from the Botanical garden of the University of Bologna was studied on platelets obtained either from aspirin-naive subjects with at least one cardiovascular risk factor or from aspirin-treated patients with a prior diagnosis of coronary artery disease (CAD). Authors demonstrated that verbascoside at a concentration of 1 mg/dL significantly inhibited platelet aggregation induced by arachidonic acid and ADP, *in vitro* (Campo G, et al. J Thromb Thrombolys. 2012;34:318-325*).* The same research group also evaluated the antiplatelet effect of verbascoside obtained from the same source in subjects with at least one cardiovascular risk factor, in a randomized single-center double-blind, phase II study. Authors demonstrated that verbascoside administered at a daily dose of 100mg for 2 weeks, significantly reduced platelet aggregation induced by arachidonic acid and ADP (Campo G, et al. Pharmacol Res. 2015,-97.-1-6*).*

However, to the best of our knowledge, the effect of Acanthus mollis seeds methanol extract on human platelet activation has not been investigated so far.

### DESCRIPTION OF THE INVENTION

In the first step of the present research protocol we aimed to identify the best method of extraction of the *Acanthus mollis* seeds in order to obtain the extract exhibiting the best biological activity towards platelet aggregation. Therefore, 13g of dry seeds (stored at -20°C, two days after their harvesting), were grinded-pulverized with a brass mortar into a powder. The powder was then extracted sequentially with 200 mL of two solvents of gradually increasing polarity first hexane and then methanol in a Soxhlet apparatus for 6h with each solvent. We ended up in this extraction method after having found in preliminary experiments that among all extracts prepared (hexane extract, ethyl acetate extract, dichloromethane extract and methanol extract) obtained by extraction in a Soxhlet apparatus only the methanol extract exhibited biological activity towards platelet aggregation, whereas all other extracts had little or no antiplatelet effect.

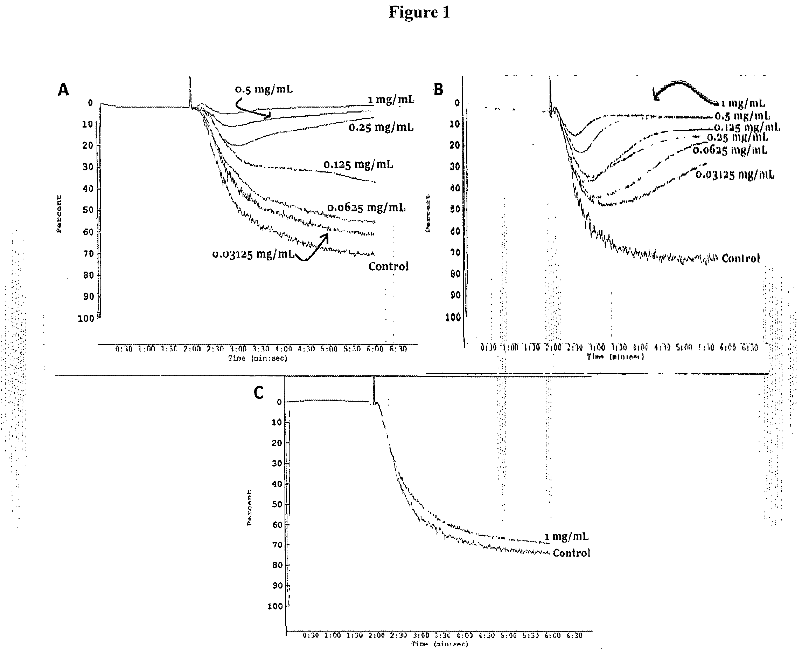

The effect of methanol extract on platelet aggregation *in vitro* was extensively studied in human Platelet Rich Plasma (PRP) from healthy volunteers, using Light Transmittance Aggregometry (LTA), as we previously described *(*Mitsios JV, et al. Eur J Biochem. 2004;271:855-862*).* PRP was activated by arachidonic acid (AA; 0.5 mM), Thrombin Receptor Activating Peptide-6 (TRAP-6; 10 µM) and ADP (10 µM), as agonists. Samples were prepared by evaporating the methanol under nitrogen and then by dissolving the residue in DMSO. The maximal aggregation, achieved within 4 min after the addition of each agonist, was determined and expressed as a percentage of 100% light transmission calibrated for each specimen (% maximal aggregation). The inhibitory efficacy of the seeds extract expressed as IC₅₀ value (half maximal inhibitory concentration). All values derived from at least three independent experiments performed for each platelet agonist, using 3 different methanol extract preparations (prepared as described above) and are expressed as the mean ± standard deviation (S.D.). The final concentration of DMSO in each assay was 0.01 % by volume. As it is shown in Figure 1, the methanol extract of *Acanthus mollis* seeds inhibits, in a dose-dependent manner, platelet aggregation induced by AA (IC₅₀ = 0.15 mg/mL) and TRAP-6 (IC₅₀ = 0.14 mg/mL), but not by ADP, indicating that its inhibitory effect concerns specific platelet activation pathways, mediated by AA (it is metabolized in platelets by COX-1 to generate TxA₂ which activates platelets via its receptor), and the platelet receptor PAR-1 of thrombin, which is specifically activated by the peptide TRAP-6 used in our experiments.

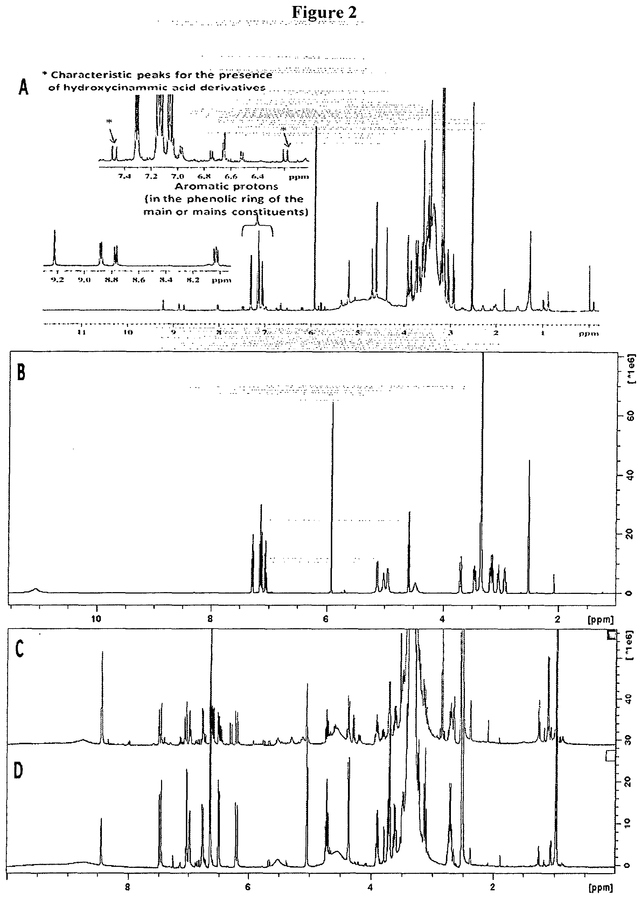

Subsequently, analysis of the phytochemical profile of the methanol extract of *Acanthus mollis* seeds was performed. The residue derived after methanol evaporation under nitrogen (5 mg) was dissolved in 0.5 mL DMSO-d₆ and transferred to NMR tubes (5 mm), so that ID ¹H-NMR spectra was recorded and characteristic peaks were indicated (Figure 2A). Trimethylsilylpropanoic sodium salt (TMSP-d4) used as a reference compound. NMR experiments held at the Center of Nuclear Magnetic Resonance of University of Ioannina, on a Brüker LC-NMR AV-500 spectrometer (Brüker). Experimental conditions were as follows: number of scan (NS) = 256, T=298 K, experimental time= 30 min, 500 MHz). The NMR system was controlled by the software Top Spin (Brüker). The assessment of results was based on 2D ¹H-¹³C HSQC and HMBC spectra.

LC-MS method was also used for the determination of the major compounds of the methanol extract as a supplementary method of the NMR spectroscopy, and vice versa. Samples from the methanol extract were diluted to reach the concentration of 0.05 mg/mL. Ten µL of filtered sample (0.45 µm) were injected into the LC-MS instrument. All LC-MS" experiments were performed on a quadrupole ion trap mass analyzer (Agilent Technologies, model MSD trap SL) retrofitted to a 1100 binary HPLC system equipped with an degasser, autosampler, diode array detector and electrospray ionization source (Agilent Technologies, Karlsruhe, Germany). All hardware components were controlled by Agilent Chemstation Software. Separation was achieved on a 15 cm x 4.6 mm i.d., 5 µm Zorbax Eclipse XDB-C18 analytical column (Agilent, USA), at a flow rate of 0.6 mL/min, using as solvent A (water/formic acid, 99.9: 0.1 v/v) and solvent B (acetonitrile). The gradient used for the analysis was: 0-6 min 90% A, isocratic elution; 6-10 min 95-85% A; 10-15 min 85% A, isocratic elution; 15-20 min 85-75% A; 20-35 min 75-60% A; 35-40 min 60-90% A. The UV/vis spectra were recorded in the range 200-550 nm and chromatograms were acquired at 280 and 330 nm.

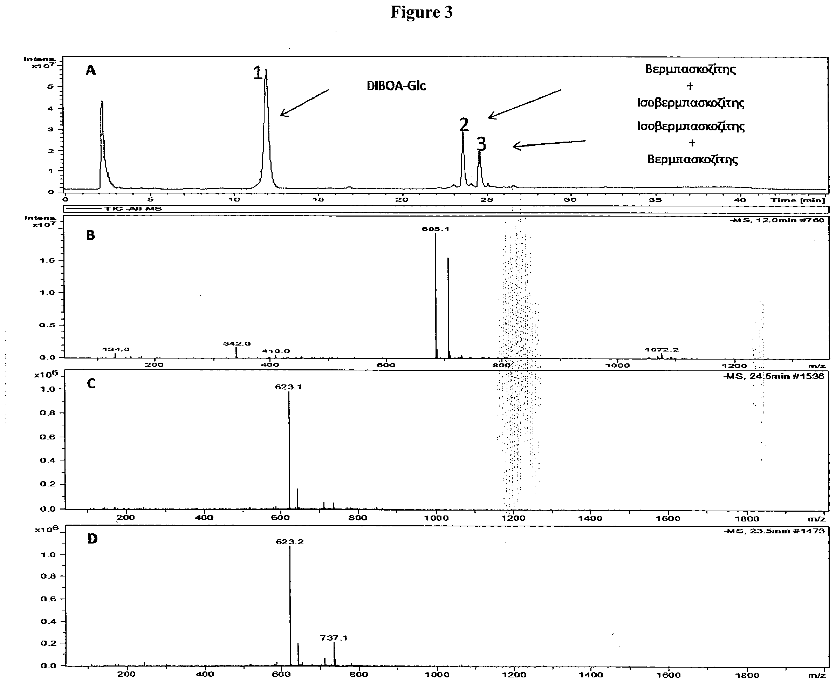

Both precursor and product (MS² and MS³) ions scanning of the phenolic compounds were monitored between *m*/*z* 50*-m*/*z* 1.200 in negative polarity. The ionization source conditions were as follows: capillary voltage, 3.5 kV; drying gas temperature, 350 °C; nitrogen flow and pressure, 11 L/min and 50 psi, respectively. Maximum accumulation time of ion trap and the number of MS repetitions to obtain the MS average spectra were set at 30 and 3 ms, respectively. The total ion current (TIC) chromatogram (Figure 3A), UV chromatogram and MS, MS² and MS³ spectra were recorded. Identification of major compounds (1, 2 and 3) was based on accurate mass measurements of the pseudomolecular [M-H]⁻ ions and their fragmentation pattern, as it has been previously described (Wolf RB, et al. J Nat Prod. 1985;48:59-63*;* Ryan D, et al. J Chromatogr. A 1999;832:87-96). According to the results, **peak 1** contains the compound 2-O-β-D-glucopyranosyl-4-hydroxy-1,4-benzoxazin-3-one (dimer) (DIBOA-Glc), **peak 2** contains mainly Verbascoside and traces of Isoverbascoside (Isoacteoside), whereas **peak 3** contains mainly Isoverbascoside and traces of Verbascoside. All characteristics of these compounds are summarized in the following table.

**Table**

| Peak | Rₜ (min) | [M-H]⁻ (*m*/*z*) | -MS² [M-H]⁻ (*m*/*z*) (%) | -MS³ [base peak]⁻ (*m*/*z*) (%) | Compounds |
|---|---|---|---|---|---|
| 1 | 12.0 | 685 (100), 342 (12) | 342 (100) | 134 (100), 180 (28), 162 (15) | DIBOA-Glc |
| 2 | 23.6 | 623 (100) | 461(100) | 135 (100), 315 (99), 161 (16), 297 (19) | Verbascoside, Isoverbascoside (in traces) |
| 3 | 24.6 | 623 (100) | 461 (100), 315 (3) | 135 (100), 315 (96), 161 (12), 297 (12) | Isoverbascoside, Verbascoside (in traces) |

In order to ascertain the identity of the major compounds, these were isolated by using preparative High Pressure Liquid Chromatography (HPLC). The residue derived after methanol evaporation under nitrogen (20-60 mg) was again dissolved in methanol (3.2 mL). 3.1 mL of filtered sample (0.45 µm) was injected into the HPLC instrument (preparative Chromatography system, *Shimadzu, Japan*) and separation was achieved on 25 cm x 21.2 mm i.d., 10 µm Ascentis C18 analytical column (*Shimadzu, Japan*), at a flow rate of 16 mL/min, using as solvent A (water/formic acid, 99.9: 0.1 v/v) and solvent B (acetonitrile). The gradient used for the analysis was the same as described in the LC-MS experiments and the UV/vis spectra were acquired at 280 and 330 nm. The fractions were lyophilized before they used in subsequent experiments.

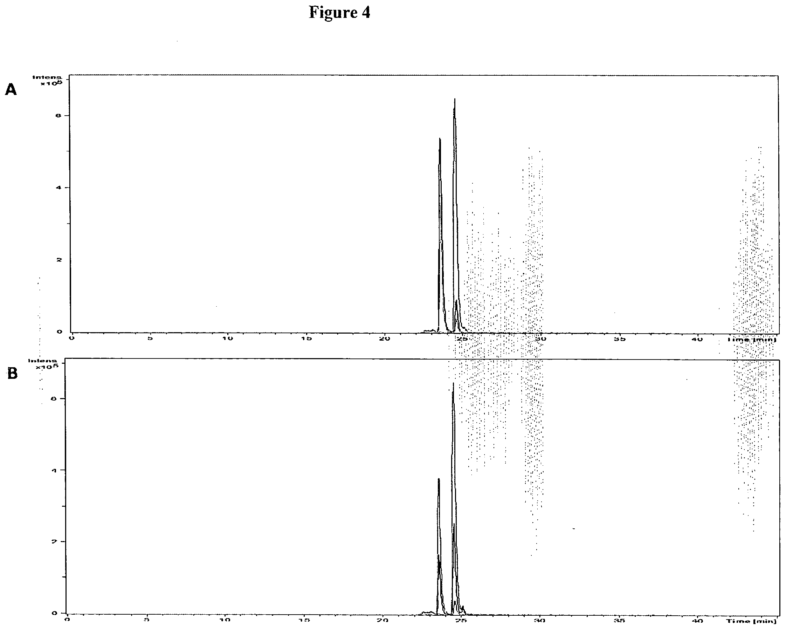

Three fractions were isolated using the above HPLC methodology and were then analyzed by LC-MS using the same experimental conditions described above. TIC chromatograms, UV chromatograms and MS, MS² and MS³ spectra were recorded. According to our results, Fraction 1 corresponds to DIBOA-Glc (Figure 3B), while Fraction 2 (Figure 3C) and Fraction 3 (Figure 3D) are a mixture of Verbascoside and Isoverbascoside, the Verbascoside been in greater quantity over Isoverbascoside in Fraction 2 and Isoverbascoside been in greater quantity over Verbascoside in Fraction 3. In order to ascertain the presence of Verbascoside and Isoverbascoside, authentic standard compounds (Medchem Express, USA) were used to the desired concentration of 0.05 mg/mL (Figure 4).

Additionally, the isolated by HPLC fractions were submitted to analysis by NMR. Each fraction (5 mg) was dissolved in 0.5 mL DMSO-d6 and transferred to NMR tubes (5 mm), so that ID ¹H-NMR spectra were recorded. Trimethylsilylpropanoic sodium salt (TMSP-d4) was used again as reference compound. The complete assignment of Fraction 1 as DIBOA-Glc (Figure 2B), Fraction 2 (Figure 2C) and Fraction 3 (Figure 2D) as a mixture of Verbascoside and Isoverbascoside was in accordance with previously published values (Hartenstein H, et al. Phytochemistry. 1994;35:827-828*.;* Kanchanapoom T, et al. Phytochemistry. 2011;58:637-640*.;* Yin H, et al. J Chromatogr. A. 2008;1205:177-181).

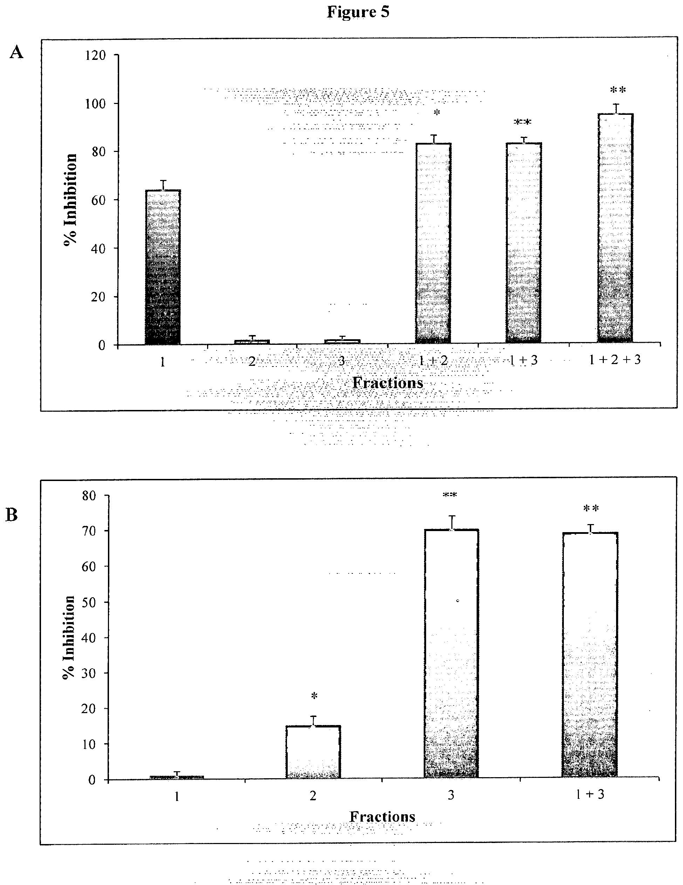

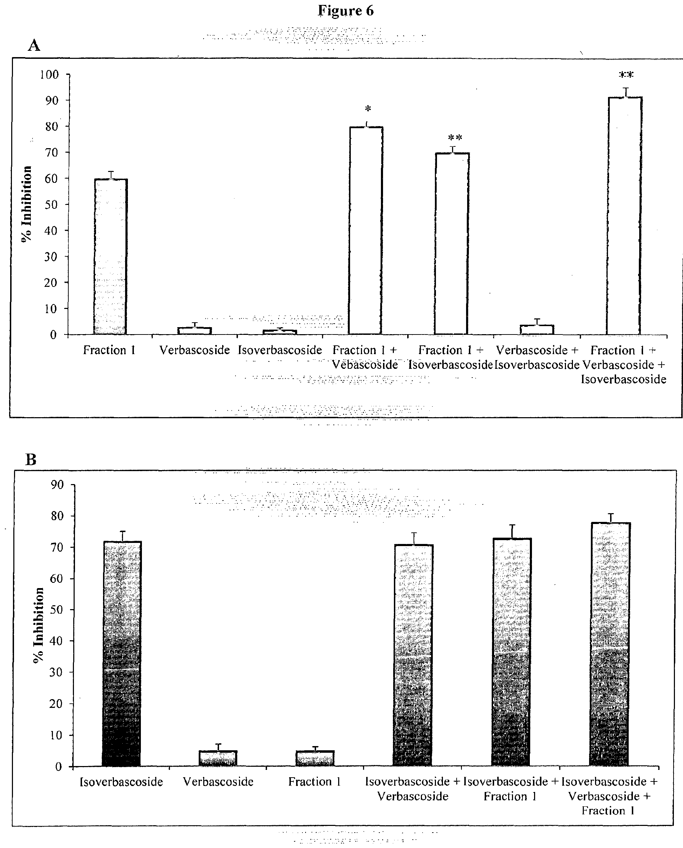

Following the above analysis, the biological effect of the three fractions towards platelet aggregation *in vitro* was studied in PRP by the LTA as we described above, using AA (0.5 mM) and TRAP-6 (10 µM) as agonists, since the total extract inhibited platelet aggregation induced only by these 2 agonists (Figure 1). Each fraction was dissolved in DMSO and used at a final concentration of 1 mg/mL. Standards of Verbascoside and Isoverbascoside were also dissolved in DMSO and used at a final concentration of 1 mg/mL. As it is shown in Figure 5A, among the 3 fractions, only fraction 1 (DIBOA-Glc) inhibited platelet aggregation induced by AA, exhibiting a threshold concentration (the lower concentration that induces the maximum inhibitory effect) of 0.2 mg/mL. This concentration was not much lower to that of the total extract 0.25 mg/mL (as it would expected, since we used a purified fraction from the total extract that expresses the inhibitory activity towards AA-induced platelet aggregation). Therefore, we used a combination of fraction 1 with fraction 2 or 3 (at mass ratios of 1 : 1) or a combination of the 3 fractions (at mass ratios of 1 : 1 : 1). As it is shown in Figure 5A, fractions 2 or 3 do not inhibit platelet aggregation, however, they significantly increased the antiplatelet effect of fraction 1, the maximum inhibition been observed when the combination of 3 fractions was used. This suggests that Verbascoside and Isoverbascoside express a synergistic inhibitory effect with DIBOA-Glc towards AA-induced platelet aggregation. To further support this suggestion, we studied the effect of standards Verbascoside and Isoverbascoside in combination with fraction 1 (DIBOA-Glc). As it is shown in Figure 6A, Verbascoside and Isoverbascoside alone or their combination do not inhibit platelet aggregation towards AA-induced platelet aggregation but significantly increased the antiplatelet effect of fraction 1 (DIBOA-Glc), the maximum inhibition been observed when their combination was added to fraction 1.

Next we evaluated the biological effect of the three fractions towards platelet aggregation induced by TRAP-6. As it is shown in Figure 5B, inhibitory activity was expressed mainly by Fraction 3 (containing primarily Isoverbascoside and traces of Verbascoside), whereas a much lower activity was express by fraction 2 (containing primarily Verbascoside and traces of Isoverbascoside). Fraction 1 had no inhibitory activity towards platelet aggregation induced by TRAP-6, neither it increased the inhibitory activity expressed by fraction 3 (Figure 5B). These results suggest that the inhibitory effect of the total methanol extract towards TRAP-6-induced platelet aggregation is primarily attributed to Isoverbascoside. To further support this suggestion, we studied the effect of standards Isoverbascoside and Verbascoside alone or their combination (at mass ratio 1 : 1) as well as in combination with fraction 1 (DIBOA-Glc) (at mass ratio 1 : 1 : 1). As it is shown in Figure 6B, only Isoverbascoside inhibited platelet aggregation, whereas Verbascoside or fraction 1 (DIBOA-Glc) or their combination, failed to increase Isoverbascoside's inhibitory effect.

Overall, the methanol extract of *Acanthus mollis* seeds, inhibits platelet aggregation mediated through AA pathway and PAR-1 receptor. The inhibitory effect towards AA is primarily attributed to its DIBOA-Glc content, whereas a synergistic effect is observed by the combination of DIBOA-Glc with Isoverbascoside, Verbascoside as well as their combination. By contrast, the inhibitory effect towards platelet aggregation mediated through PAR-1 receptor is exclusively attributed to its Isoverbascoside content.

To date, patients with cardiovascular disease should receive antiplatelet therapy, primarily aspirin (inhibits COX-1 and therefore AA-induced platelet aggregation) and an ADP receptor P2Y12 antagonist (clopidogrel, prasugrel or ticagrelor) (Kalantzi KI, et al. Expert Rev Clin Pharmacol. 2012;5:319-36*;* Tsoumani ME, Tselepis AD, Curr Pharm Des. 2017*; Epub ahead of print*)). Several studies however have demonstrated that some patients receiving aspirin or clopidogrel do not adequately respond to the drug action and exhibit a new ischemic cardiovascular event (a phenomenon named as aspirin or clopidogrel resistance). Recent studies showed that a PAR-1 antagonist (vorapaxar) exhibit an important clinical efficacy in preventing an atherothrombotic event, however its therapeutic usefulness has been limited due to the increase in bleeding risk (Moschonas IC, et al. Int J Cardiol. 2015;185:9-18).

Therefore, based on the present results, the constituents of *Acanthus mollis* seeds described above may be considered as new therapeutic agents to be used in CVD patients, since they can replace aspirin in aspirin resistant patients (DIBOA-Glc alone or in combination with Verbascoside or/and Isoverbascoside) and also to be used as a specific antagonist of the platelet thrombin receptor PAR-1 (Isoverbascoside). Finally, the total extract could be used as an important supplement in addition to the standard therapy with aspirin and a P2Y12 antagonist in these patients.

### BRIEF DESCRIPTION OF SHEMES

**Figure 1** shows representative dose-response curves for the inhibitory effect of the methanol extract of *Acanthus mollis* seeds on platelet aggregation induced by A.A (0.5 mM) **(A)** and TRAP-6 (10 µM) **(B)** but not by ADP (10 µM) **(C).** The final concentration of DMSO was 0.01 % by volume.
**Figure 2** shows the ID ¹H-NMR spectrum of the methanol extract of *Acanthus mollis* seeds (A), fraction 1 **(B),** fraction 2 **(C)** and fraction 3 **(D)** in DMSO-*d*₆ (NS= 256, T=298K, experimental time = 30 min, 500 MHz). As it can be observed in figures 2C and 2D fractions 2 and 3 contain a mixture of Verbascoside and Isoverbascoside.
**Figure 3** shows **(A)** the TIC chromatogram of *A. mollis* methanolic extract where the main components are indicated, **(B)** the MS spectrum of fraction 1 (m/z 685.1, Rₜ=11.9 min), **(C)** the MS spectrum of fraction 2 (m/z 623.2, Rt=23.6 min) and **(D)** the MS spectrum of fraction 3 (m/z 623.2, Rt=24.5 min).
**Figure 4** shows an overlaid total ion chromatograms of **(A)** fraction 2 (black), standard Verbascoside (red) and Isoverbascoside (green), demonstrating the existence of verbascoside and a small quantity of isoverbascoside in fraction 2 and (B) fraction 3 (black), standard Verbascoside (red) and Isoverbascoside (green), demonstrating the existence of Isoverbascoside and a small quantity of Verbascoside in fraction 3.
**Figure 5** is a bar-graph demonstrating the effect of fractions 1,2 and 3 as well as their combinations on human platelet aggregation in PRP induced by **(A)** AA (0.5 mM) and **(B)** TRAP-6 (10 µM). The final concentration of each fraction in PRP was 1 mg/mL. The final concentration of DMSO in PRP was 0.01 % by volume. * P < 0.05 and ** P < 0.01 compared with fraction 1.
**Figure 6** is a bar-graph demonstrating the effect of fraction 1, Verbascoside and Isoverbascoside as well as their combinations on human platelet aggregation in PRP induced by **(A)** AA (0.5 mM) and **(B)** TRAP-6 (10 µM). The final concentration in PRP of each agent used was 1 mg/mL. The final concentration of DMSO was 0.01 % by volume. * P < 0.05 and ** P < 0.01 compared with fraction 1.

## Claims

1. A methanolic extract of *Acanthus mollis* seeds for use in the inhibition of human platelet aggregation.

2. 2-O-β-D-glucopyranosyl-4-hydroxy-1,4-benzoxazin-3-one (dimer) (DIBOA-Glc) for use in the inhibition of human platelet aggregation.

3. Isoverbascoside for use in the inhibition of human platelet aggregation.

4. A combination of 2-O-β-D-glucopyranosyl-4-hydroxy-1,4-benzoxazin-3-one (dimer) (DIBOA-Glc) with Isoverbascoside or Verbascoside or both for use in the inhibition of human platelet aggregation.

## Patentansprüche

1. Methanolischer Extrakt von *Acanthus mollis-Samen* zur Verwendung bei der Hemmung der menschlichen Thrombozytenaggregation.

2. 2-O-β-D-Glucopyranosyl-4-hydroxy-1,4-benzoxazin-3-on (Dimer) (DIBOA-Glc) zur Verwendung bei der Hemmung der menschlichen Thrombozytenaggregation.

3. Isoverbascosid zur Verwendung bei der Hemmung der menschlichen Thrombozytenaggregation.

4. Kombination von 2-O-β-D-Glucopyranosyl-4-hydroxy-1,4-benzoxazin-3-on (Dimer) (DIBOA-Glc) mit Isoverbascosid oder Verbascosid oder beidem zur Verwendung bei der Hemmung der menschlichen Thrombozytenaggregation.

## Revendications

1. Extrait méthanolique de graines d'*Acanthus mollis* pour son utilisation dans l'inhibition de l'agrégation plaquettaire humaine.

2. 2-O-β-D-glucopyranosyl-4-hydroxy-1,4-benzoxazin-3-one (dimère) (DIBOA-Glc) pour son utilisation dans l'inhibition de l'agrégation plaquettaire humaine.

3. Isoverbascoside pour son utilisation dans l'inhibition de l'agrégation plaquettaire humaine.

4. Combinaison de 2-O-β-D-glucopyranosyl-4-hydroxy-1,4-benzoxazin-3-one (dimère) (DIBOA-Glc) avec de l'isoverbascoside ou du verbascoside ou les deux pour son utilisation dans l'inhibition de l'agrégation plaquettaire humaine.
